(19) Europäisches Patentamt
European Patent Office
Office européen des brevets

(11) Publication number: **0 156 771 A2**

(12) **EUROPEAN PATENT APPLICATION**

(21) Application number: 85810122.3

(22) Date of filing: 22.03.85

(51) Int. Cl.[4]: C 07 D 501/06
//C07D277/74

(30) Priority: 29.03.84 AT 1055/84
24.07.84 AT 2386/84
10.09.84 AT 2884/84

(43) Date of publication of application:
02.10.85 Bulletin 85/40

(84) Designated Contracting States:
AT BE CH DE FR GB IT LI LU NL SE

(71) Applicant: BIOCHEMIE Gesellschaft m.b.H.
A-6250 Kundl Tirol(AT)

(72) Inventor: Prager, Bernhard Christian
Steinbacherstrasse 1
A-6300 Wörgl(AT)

(72) Inventor: Thaler, Heinrich
A-6322 Kirchbichl 195(AT)

(72) Inventor: Sturm Hubert
Urichstrasse 102
A-6500 Landeck(AT)

(74) Representative: Norris, Allen Edwin et al,
SANDOZ AG Patentabteilung
CH-4002 Basel(CH)

(54) **Cephalosporins.**

(57) A process for the preparation of compounds of formula I

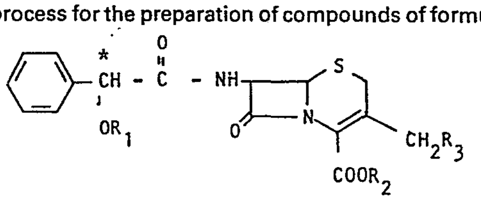

I

wherein $R_1$ represents hydrogen or a hydroxy-proctecting group,

$R_2$ represents hydrogen, a cation, the pivaloyloxymethyl group or a carboxy-protecting group and

$R_3$ represents hydrogen, the acetoxy group, the carbamoyloxy group, a group S–Y whereby Y represents an unsubstituted or substituted heterocycle, or an optionally substituted pyridinium of formula

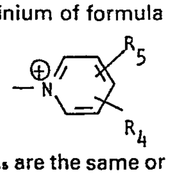

II

whereby $R_4$ and $R_5$ are the same or different and each represent hydrogen, halogen, alkyl, hydroxy, carboxamido, alkoxycarbonyl amino, monoalkylamino or di-alkylamino or together represent an optionally substituted 5- or 6-membered carbocyclic ring, characterised by reacting a compound of formula IV

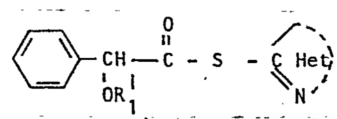

IV

wherein $R_1$ represents a hydroxy protecting group and

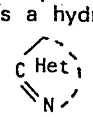

represents a 5- or 6-membered heterocycle which may contain in addition to the nitrogen atom one or two further heteroatoms and which may be substituted or anellated with an optionally substituted benzene ring with a compound of formula III and if desired deprotecting the obtained end product and optionally converting an obtained product into a salt or vice-versa.

EP 0 156 771 A2

CEPHALOSPORINS

The invention concerns a novel process for the preparation of compounds of formula

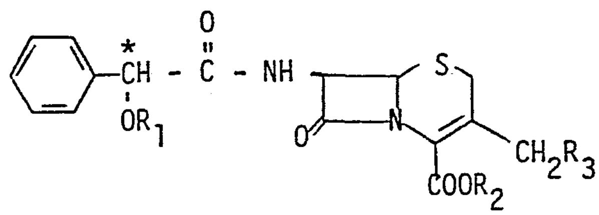

I

wherein $R_1$ represents hydrogen or a hydroxy-protecting group,
$R_2$ represents hydrogen, a cation, the pivaloyloxymethyl group or a carboxy-protecting group and
$R_3$ represents hydrogen, the acetoxy group, the carbamoyloxy group, a group S-Y whereby Y represents an unsubstituted or substituted heterocycle, or an optionally substituted pyridinium of formula

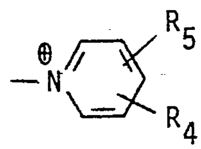

II

whereby $R_4$ and $R_5$ are the same or different and each represent hydrogen, halogen, alkyl, hydroxy, carboxamido, alkoxycarbonyl amino, monoalkylamino or di-alkylamino or together represent an optionally substituted 5- or 6-membered carbocyclic ring.

The compounds of formula I represent a known class of cephalosporin antibiotics (e.g. Cefamandole, Cefamandole nafat, Cefonicid) which is to a large extent described in patents and publications as well as protected forms thereof.

In these structures $R_3$ may be hydrogen. It can also be carbamoyloxy. However it preferably represents acetoxy or S-Y. Suitable heterocylces which Y may represent are known, for example, from numerous publications. Preferred heterocycles are for example thiadiazolyl, diazolyl, triazolyl, tetrazolyl, thiazolyl, thiatriazolyl, oxazolyl, oxadiazolyl, triazolylpyridyl, purinyl, pyridyl, pyrimidinyl, pyridazinyl, pyrazolyl and triazinyl. These heterocycles may be unsubstituted or for example up to

tri-substituted. Suitable substituents include $(C_{1-4})$alkyl, $(C_{1-4})$alkoxy, halogen, trihalo-$(C_{1-4})$alkyl, hydroxy, oxo, mercapto, amino, carboxyl, carbamoyl, di-$(C_{1-4})$alkylamino, carboxymethyl, carbamoylmethyl, sulfomethyl and methoxycarbonylamino. Heterocycles mentioned in the literature as particularly preferred include tetrazolyl, especially 1-methyl-1H-tetrazol-5-yl, and 1-sulpho methyl-1H-tetrazol-5-yl. $R_3$ is preferably acetoxy, 1-methyl-1H-tetrazol-5-yl or 1-sulphomethyl-1H-tetrazol-5-yl.

As is known in the cephalosporin field the compounds can be in the form of the free acids or of salts, for example of alkali- and alkaline earth-metal salts especially alkali metal salts such as sodium salts. Furthermore the compounds can also be in the form of esters, for example in the form of the pivaloyloxymethyl ester ($R_2$ = pivaloyloxymethyl). Other carboxy protecting groups which $R_2$ may represent are generally known and include for example acetoxymethyl, 1-acetoxyethyl, 1-ethoxycarbonyloxyethyl, 5-indanoyl or preferably hexanoylmethyl, phthalidyl, carbethoxymethoxymethyl, 3-carbethoxy-1-acetonyl. or trialkylsilyl, particularly trimethylsilyl.

Compounds of formula I possess in the acylamino side chain in position 7 ofthe cephem nucleus an asymmetric carbon atom and may therefore be present in D- or L-Form. It is an often observed fact in cephalosporin chemistry that only one of the two epimeric forms possesses the desired antibacterial activity.

In the case of the compounds herein it is the derivatives of D-(-)-mandelic acid which are preferred.

As mentioned, the compounds of formula I are generally known, and various methods for their preparation have already been proposed, one such method comprises acylation of the corresponding, optionally protected 7-aminocephalosporanic acid derivative of formula

Case 900-9721/WA

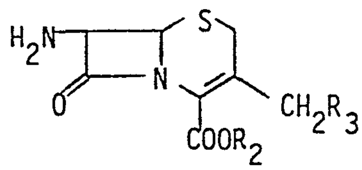

III

wherein $R_2$ and $R_3$ have the above meaning, with an activated form of the corresponding D-(-)-mandelic acid derivative. Such acylation methods are known and described e.g. in AT-Patent Specification 330,347 and US-Patent Specification 3,641,021.

Anhydro-O-carboxymandelic acid of the formula

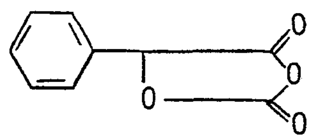

and o-formylmandelic acid chloride are for example employed hereby as reactive derivatives of the mandelic acid. This form of activation has however the great disadvantage that when preparing the activated derivative with phosgen or oxalylchloride, hydrochloric acid is liberated. It is known that optically active carbonic acids tend to racematise in the presence of strong acids. This results in the activated form prepared starting from the corresponding D-(-)-mandelic acid derivative also containing a share of the epimeric L-(+)-derivative. The acylation of compounds of formula III with these activated forms of mandelic acid therefore produces compounds of formula I which contain a share of the undesired L-(+) derivative.

This partial racematisation can be demonstrated for example by reconversion of the active derivative under mild conditions to the starting material e.g. by hydrolysis using $NaHCO_3$ with ice-cooling. Such recovered mandelic acid possesses a rotation value of $[\alpha]_D^{20} \leq -150°C$ ($H_2O$, c = 2,5) whereas pure D-(-)-mandelic acid measured under the same conditions has a rotation value of -158°. This corresponds to an amount of $\geq$ 2% of L-(+)-mandelic acid in the regenerated product.

The present invention provides a process whereby the desired compound of formula I can be obtained in high yield and purity.

In particular the present invention concerns a process for the preparation of compounds of formula I and their salts characterised by reacting a compound of formula IV

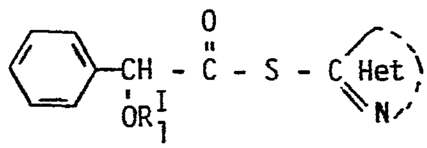

IV

wherein $R_1^I$ represents a hydroxy protecting group and 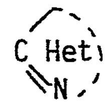 represents a 5- or 6-membered hererocycle which may contain in addition to the nitrogen atom one or two further heteroatoms, for example O,N and S, and which may be substituted or anellated with an optionally substituted benzene ring with a compound of formula III and if desired deprotecting the obtained end product and optionally converting an obtained product into a salt or vice-versa. The optical activity is fully retained as the process may be run under extremely mild and practically neutral conditions.

The process is suitably carried out in a solvent inert under the reaction conditions e.g. in a chlorinated hydrocarbon such as dichloromethane, or an ester such as ethylacetate or in mixtures of such solvents. The reaction temperature is suitably from -40 to +60°C, especially -15 to +25°C and preferably 0 to 20°C and the reaction time can vary between 1/2 to 48 hours. The reactants of formulae III and IV can be used in stoichiometric amounts. On the other hand an excess of up to 25% of the compound of formula IV may be employed.

When preparing compounds wherein $R_2$ represents hydrogen (as well as their salts) it may be appropriate to protect the carboxyl group in the starting material. Suitable protecting groups are known and include not only the above mentioned possible meanings for $R_2$ but also silylester protecting groups, especially the trimethyl silyl protecting groups which

may be introduced e.g. by reaction of the free acid with N,O-bistrimethylsilylacetamide.

After reaction of the compounds of formulae III and IV any subsequent deprotection reactions can be carried out in known manner.

One of the hydroxy protecting groups preferably used in the process is the formyl group. In order to remove this formyl group it is usual to saponify in aqueous, weakly alkaline solution e.g. as described in DE-Patent Specification 2,018,600. This results in the partial formation of by-products and usually leads to a dark colouration of the reaction solutions. Furthermore lengthy reaction times and temperatures above room temperature are usually required. Cephalosporin derivatives are known to be unstable in the alkaline range. Even in weakly alkaline media (pH-value: 8-9) such as used in the saponification of formyl protected compounds of formula I they are significantly less stable than in the weakly acidic range.

The invention also provides a method of deprotecting such formyl derivatives involving a mild interchange of the ester radical in the formic acid ester with an aliphatic alcohol of formula V

$$HO\text{-}R_6 \qquad V$$

wherein $R_6$ stands for a lower, straight chain or branched alkyl group. The process is suitably carried out in acid media under mild conditions as shown in the following reaction scheme

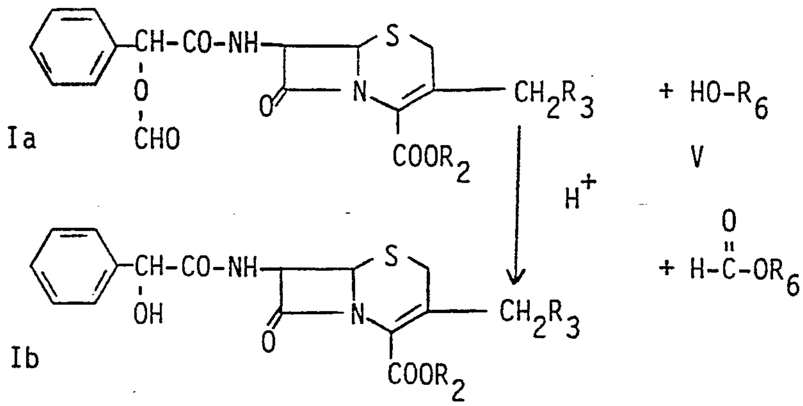

Ia

V

Ib

This deprotection according to the invention enables rapid, careful and practically quantative preparation of compounds of formula I, wherein $R_1$ represents hydrogen from compounds of formula I wherein $R_1$ stands for the formyl group. By virtue of the mild conditions in acid media almost no by-product- or pigment-formation occurs, representing a considerable advantage over the alkaline saponification as described in DE-Patent Specification 2,018,600.

This deprotection can for example be carried out by dissolving a formyl protected compound of formula Ia in a alcohol of formula V, treating with an acid and under these conditions preparing a compound of formula Ib. The reaction usually takes place in an excess of the alcohol of formula V used as reaction partner as solvent. Suitable alcohols of formula V are aliphatic alcohols with 1 to 4 carbon atoms whereby the aliphatic chain may be branched. Preferred alcohols are methanol, ethanol and isopropanol whereby methanol is preferably employed. Strong organic or mineral acids may be used as acids for the catalysis of the ester exchange. The preferred acid is concentrated hydrochloric acid. The reaction is preferably carried out using an acidic ion exchanger, for example DOWEX 50 WX 4 or 8. The temperature of the ester exchange is not critical and suitably lies between -10 and +50°C, preferably room temperature.

The compounds of formula IV are new. The nature of the ring 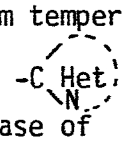 is not critical and is decided on the basis of factors such as ease of preparation and availability of starting materials. The ring preferably represents 2-p ridyl or in particular 2-benzthiazolyl. It can be also represent pyrimidinyl, triazolyl or thiazolyl.

Alternative protecting groups $R_1^I$ include trialkylsilyl, particularly trimethylsilyl protecting groups.

The compounds of formula IV are prepared by reacting a compound of formula

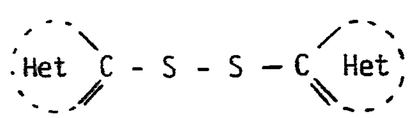 VI

wherein 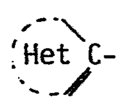 has the above meaning with a compound of formula VII

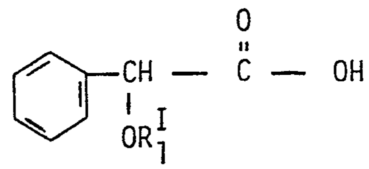

VII

wherein $R_1^I$ has the above meaning.

The process is preferably carried out in the presence of a tri (lower alkyl)- or tri(aryl)phosphine or phosphite and a tertiary organic amide such as triethylamine or N-methyl morpholine whereby the latter can be employed in a equivalent quantity to the compound of formula VI to assure neutral reaction conditions. Inert solvents not containing hydroxy groups such as dichloromethane chloroform, acetonitrile or dimethylformamide are suitable. The preparation takes place at temperatures from -50 to +50°C preferably -20 to +25°C.

It is often the case, particularly when employing protecting groups sensitive to hydrolysis e.g. the trialkylsilyl protecting group that isolation of the activated mandelic acid derivative of formula IV is unnecessary. These may preferably be reacted in situ with the corresponding 7-aminocephem of formula III. On completion of the acylation the desired products of formula I may be isolated in accordance with known methods.

The compounds of formula I are, as already mentioned, generally known antibiotics or protected forms thereof. In particular these antibiotics are indicated for use as antibacterial agents as shown in investigations in vitro using the series dilution test at a concentration of e.g. from 0.01 to 50 ug/ml and in vivo at a dose of 0.1 to 100 mg/kg body weight in mice against a varienty of strains e.g. Staphylococcus aureus, Streptococcus pyogenes, Streptococcus faecalis, E. coli, Proteus vulgaris, Proteus mirabilis, Proteus morganii, Shigella dysenteria, Shigella sonnei, Shigella flexneri, Alcaligenes faecalis, Klebsiella aerogenes, Klebsiella pneunomiae, Serrata marcescens, Salmonella Heidelberg, Salmonella typhimurium, Salmonella enteritidis and Nesseria gonorrhoae.

The antibiotics are therefore useful as anti-bacterial agents. For this use the dose to be administered depends on the compound used and the mode of administration as well as th nature of the treatment. Satisfactory results are obtained by administration of a daily dosage of ca. 1 to 6 g conveniently administered in correspondingly divided doses of 0.25 to 3 g two to four times daily or i retard form.

The following examples illustrate the invention whereby all temperatures are given in °C.

EXAMPLE 1 : 7-(D-2-Hydroxy-2-phenyl)acetamido-3-[(1-methyl-1H-tetrazol-5-yl)thiomethyl]-3-cephem-4-carboxylic acid.sodium salt (Cefamandole-Na)

8.4 g of D-(-)-mandelic acid are treated in 100 ml of dry dichloromethane with 12 g of N,O-bistrimethylsilylacetamide and stirred for 30 minutes at room temperature. The obtained solution of O-trimethylsilyl-D-(-)-mandelic acid is cooled to -15°. 17.4 g of triphenylphosphi 22g of bis-benzthiazol-2-yldisulfide and 5g of N-methylmorpholine are ad and stirred for 1 hour at -15° to -10°. 14.8g of 7-amino-3-[(1-methyl-1 tetrazol-5-yl)thiomethyl]-3-cephem-4-carboxylic acid are suspended in 100 ml of dry dichloromethane. 10.2 g of N,O-bistrimethylsilylacetamide are added and stirred at room temperature until a clear solution results This solution is cooled to -15° and added to the active ester solution.

Stirring is continued for 2 hours at -10° and then overnight at 0°. The mixture is largely evaporated on a Rotavapor and the oily residue dissolved in 200 ml of prewarmed Isopropanol at 40°, 50 ml of a mono-molar solution of sodium methylhexanoate in isopropanol is added and cooled to 20° whereupon the final product begins to precipitate. After 1 hour the mixture is cooled to 0° and stirred for 2 hours to increase the yield. The product is filtered off washed with isopropanol and dried at 40° under vacuum. 19.4 g of a white crystalline powder (89%) are obtained. According to the TLC-method of USP XX the product is identical with a USP-Standard.

EXAMPLE 2 : 7-(D-2-Formyloxy-2-phenyl)acetamido-3-[(1-sulfomethyl-1H-tetrazol-5-yl)thiomethyl]-3-cepham-4-carboxylicacid.disodium salt

9.6 g of Bis-benzthiazol-2-yldisulfide and 7.6 g of triphenylphosphine are dissolved in 50ml of dichloromethane cooled to -15°. 5.2 of O-Formyl-D-(-)-mandelic acid are added and stirred for 2 hours at -15°. 10.5 g of 7-amino-3-[(1-sulfomethyl-1H-tetrazol-5-yl)thiomethyl]-3-cephem-4-carboxylic acid.sodium salt in 25 ml of dry dimethylformamide are treated with 3.25 ml of triethylamine, whereby a clear solution results which is added to the above mentioned solution taking care that the internal temperature does not rise above 0°. Stirring is carried out for 12 hours at 0° and then 4 hours at room temperature. The dichloromethane is removed on a Rotavapor and the viscous residue dissolved in 250 ml of isopropanol previously treated with 40 ml of a mono-molar solution of sodium ethyl hexanoate. Stirring is continued for 2 hours at 0°, followed by filtration and washing with isopropanol. 13.5 g (93.3%) of the title compound are obtained as a white powder.

NMR: 8.30 (s) formylproton; 7.35 (m) phenylprotons; 6.05 (s) benzylproton; 5.5 (d) $H_7$; 5.0 (s) $CH_2$-$SO_3$; 4.85 (d) $H_6$; 4.30 (d) $CH_2$ (exo); 3.45 (d) $CH_2$ (ring).

EXAMPLE 3: 7-(D-2-hydroxy-2-phenyl)acetamido-3-[(1-sulfomethyltetrazol-5-yl)thiomethyl]-3-cephem-4-carboxylic acid.disodium salt (Cefonicid)

11.8 g of 7-(D-2-formyloxy-2-phenyl)acetamido-3-[(1-sulfomethyl-1H-tetrazol-5-yl)thiomethyl]-3-cephem-4-carboxylic acid.monosodium salt are dissolved in 100 ml of methanol and treated with 2 ml of conc. HCl. The solution is maintained at 30° for 30 minutes whereby quantative de-formylation takes place. The pH value of the reaction mixture is then fixed to 2.5 with 5N NaOH and the reaction mixture then concentrated under vacuum to some 50 ml, filtered and then dropped with thorough stirring into 700 ml of isopropanol. The precipitated solids are filtered off, washed with isopropanol and dried under vacuum. 10.9 g of the title product are thus obtained with a water content of some 4% (yield (93%).

The mono-sodium salt employed as starting material can be obtained from the disodium salt as follows.

10 g of the disodium salt (prepared for example according to Example 2) are suspended in 100 ml of dry acetone and treated with 20 g of DOWEX 50 WX8 which had previously been washed with dry acetone. Stirring is carried out for 1 hour at room temperature followed by filtering off of the ion exchanger. The clear solution after initial content determinatio with HPLC is treated with one equivalent of a mono-molar solution of sodium ethylhexanoate in isopropanol, whereupon the mono-sodium salt precipitates To increase yield a further 100 ml of isopropanol are added.

EXAMPLE 4: 7-(D-2-formyloxy-2-phenyl)-acetamido-3-[(1-methyl-1H-tetrazol-5-yl)-thiomethyl]-3-cephem-4-carboxylic acid.mono sodium salt (Cefamandolenafat)

9 g of O-formyl-D-(-)-mandelic acid are dissolved in 100 ml of dry dichloromethane and cooled to -15°. 15.7 g of triphenylphosphine, 20 g of bis benzthiazol-2-yl disulfide and 5 g of triethylamine are added and stirred for 1 hour at -15°. 14.8 g of 7-amino-3-[(1-methyl-1H-tetrazol-5-yl)thiomethyl]-3-cephem-4-carboxylic acid are suspended in 100 ml of dry dichloromethane. 10.2 g of N,O-bistrimethylsilylacetamide are added and stirred at room temperature until a clear solution results. This

solution is cooled to 15° and then added to the solution of mandelic acid active ester. Stirring is carried out for 1 hour at -10° and then 12 hours at 0°. The mixture is largely evaporated on a Rotavapor at 20° and the oily residue dissolved in 200 ml of isopropanol. 50 ml of a mono-molar solution of sodium ethylhexanoate are added whereby precipation of the end product begins towards completion. After 2 hours stirring at 0°C the product is isolated by filtration, washed with cold isopropanol and dried. 21.6 g (93.3%) of the title compound are obtained in the form of a white crystalline powder.

IR, UV and NMR correspond to data in the literature.

EXAMPLE 5: 7-(D-2-hydroxy 2-phenyl)acetamido-3--[(1-sulfomethyltetrazol-5-yl)thiomethyl]-3-cephem-4-carboxylic acid.sodium salt

19.3 g of 7-amino-3-[(1-sulfomethyltetrazol-5-yl)thiomethyl]-3-cephem-4-carboxylic acid.sodium salt in 50 ml of dry dimethylformamide are treated with 6.7 ml of triethylamine whereby a clear solution results which is then added to a solution of 16.5 g of O-formyl-D-(-)-thiomandelic acid-S-(benzthiazol-2-yl)-ester in 100 ml of dichloromethane. Stirring is carried out at 0° for 3 hours and 5 hours at 20°. The dichloromethane is removed on a Rotavapor and the viscous residue stirred into 300 ml of isopropanol whereby the O-formylcefonicid precipitates as Na-triethylamine salt. For removal of the formyl radical the intermediate is dissolved in 200 ml of methanol and treated with 30 g of DOWEX 50 WX8, a strongly acidic ion exchanger in the $H^+$-form. Stirring is carried out for 1 hour at 20° followed by filtering off of the ion-exchanger. The reaction mixture is concentrated under vacuum to some 50 ml and treated with 60 ml of a mono-molar solution of sodium ethyl hexanoate in isopropanol and then with 300 ml of isopropanol whereby the end product crystallises out. 23 g (90.5%) of a white crystalline powder are obtained.

EXAMPLE 6: 7-(D-2-hydroxy-2-phenyl)acetamido-3-[(1-methyl-1H-tetrazol-5-yl)thiomethyl]-3-cephem-4-carboxylic acid.sodium salt (Cefamandole-Na)

9.8 g of cefamandolenafat are dissolved in 60 ml of methanol. This solution is treated at room temperature under thorough stirring with 1 ml conc.HCl. The solution is then left to stand for 40-50 minutes whereby quantative de-formylation occurs. The isolation of the Cefamandole thus formed can be carried out in known manner e.g. as described in DE-Patent Specification 2,018,600. For this the reaction mixture is treated with a solution of 3 g of Na-acetate in 30 ml of methanol and the Na salt of the Cefamandole separated by precipitation with 250 ml isopropanol. 8.8g of the title compound are obtained in the form of a white powder (91% yield) the spectroscopic data of which correspond to the literature.

EXAMPLE 7: 7-(D-2-hydroxy-2-phenyl)acetamido-3-[(1-methyl-1H-tetrazol-5-yl)thiomethyl]-3-cephem-4-carboxylic acid.sodium salt (Cefamandole-Na)

4.9 g of O-formylcefamandole are dissolved in 30 ml of methanol. 5g of DOWEX 50 WX 4 (a strongly acid ion exchanger in the $H^+$ form) are added and stirred for 1 hour at 20° whereupon, according to TLC-control, de-formylation is complete. The ion exchanger is filtered off and washed thoroughly with a little methanol and the combined filtrates concentrated to 20 ml on a Rotavapor. By addition of 11 ml of a mono-molar solution of sodium ethyl hexanoate in isopropanol the sodium salt of Cefamandole precipitates. To increase yield further 50 ml of isopropanol are added (yield 93%).

EXAMPLE 8 : O-formyl-D-(-)-thiomandelic acid-S(benzthiazol-2-yl)ester (compound of formula IV)

9g of O-formyl-D-(-)-mandelic acid are dissolved in 100 ml of dichloromethane and cooled to -15°. 15.7g of triphenylphosphine, 20 g of bis benzthiazol-2—yldisulfide and 5g of trimethylamine are added and stirred for 1 hour at -15° whereby a yellow coloured solution results.

The mixture is evaporated to dryness on a Rotavapor at 20° and the oily residue triturated with 100 ml of dry diethylether. The deposited precipitate which comprises 2-mercaptobenzthiazole and triphenylphosphinic oxide is removed by filtration and the filtrate evaporated to dryness. The residue is dissolved as quickly as possible in a little warm methanol and the solution cooled to -10°. The precipitate is filtered off after 1 hour and for analytical purposes again recrystallised from methanol. O-Formyl-D-(-)-thiomandelic acid-S-(benzthiazol-2-yl)ester is obtained in the form of weakly yellow crystals, m.p. 64-67°.

NMR: 825 (s) formyl proton; 7.9 (m) o,o'-protons of the phenyl radical; 7.45 (m) m,m,p-protons of the phenyl radical and protons of the anellated phenyl ring; 6.45 (s) benzyl proton.

Evaluation of optical purity

A solution of the active ester in water/ethanol (1/1) is treated under ice cooling with one equivalent of sodiumbicarbonate and stirred for 2 hours at 0°. The precipitated mercapto benzthiazol is filtered off and the filtrate gently concentrated. The residue is suspended in ether and treated with cooling with one equivalent of 50% formic acid. The etheric phase is separated, washed with NaCl solution and concentrated. The mandelic acid thus obtained showed a rotation value of 158.5° and is thus optically pure.

CLAIMS

1. A process for the preparation of compounds of formula I

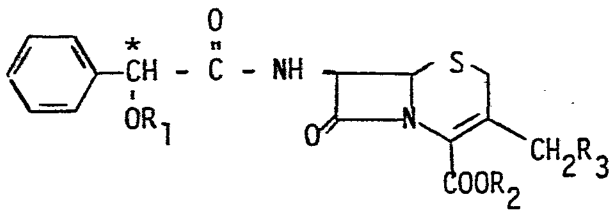

I

wherein $R_1$ represents hydrogen or a hydroxy-protecting group,
$R_2$ represents hydrogen, a cation, the pivaloyloxymethyl group or a carboxy-protecting group and
$R_3$ represents hydrogen, the acetoxy group, the carbamoyloxy group, a group S-Y whereby Y represents an unsubstituted or substituted heterocycle, or an optionally substituted pyridinium of formula

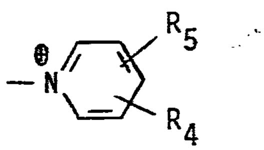

II

whereby $R_4$ and $R_5$ are the same or different and each represent hydrogen, halogen, alkyl, hydroxy, carboxamido, alkoxycarbonyl amino, monoalkylamino or di-alkylamino or together represent an optionally substituted 5- or 6-membered carbocyclic ring,

characterised by reacting a compound of formula IV

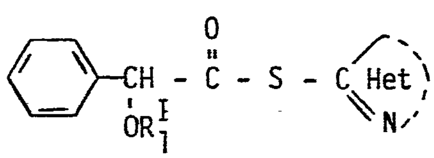

IV

wherein $R_1^I$ represents a hydroxy protecting group and 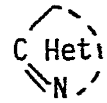 represents a

5- or 6-membered heterocycle which may contain in addition to the nitrogen atom one or two further heteroatoms and which may be substituted or anellated with an optionally substituted benzene ring with a compound of formula III and if desired deprotecting the obtained end product and optionally converting an obtained product into a salt or vice-versa.

2. A process according to Claim 1 wherein $R_1$ represents formyl.

3. A method of preparing a compound of formula Ib

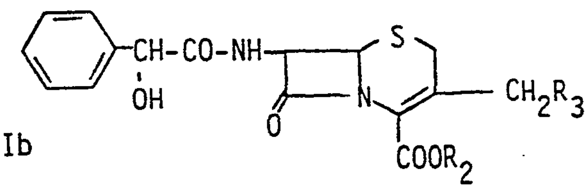

Ib

Ib

which comprises deprotecting a compound of formula Ia

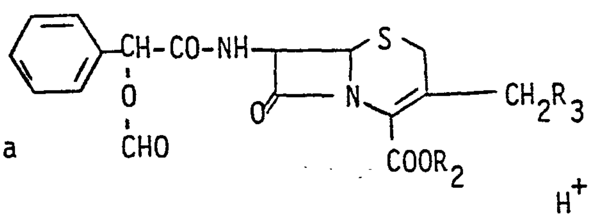


Ia

Ia

by treating with an aliphatic alcohol of formula V

$$HO - R_6 \qquad V$$

under acid conditions whereby $R_2$ and $R_3$ are as defined in Claim 1 and $R_6$ stands for lower, straight chain or branched alkyl.

4. Compounds of formula IV

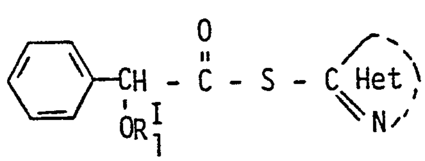

IV

wherein $R_1^I$ represents a hydroxy protecting group and 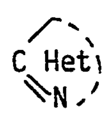 represents a

5- or 6-membered heterocycle which may contain in addition to the nitrogen atom one or two further heteroatoms and which may be substituted or anellated with an optionally substituted benzene ring.

5. A process for preparing a compound of formula IV as defined in Claim 4 which comprises reacting a compound of formula

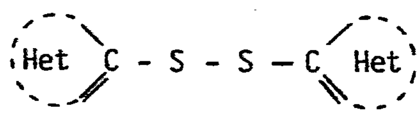

VI

wherein 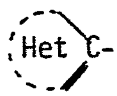 has the above meaning with a compound of formula VII

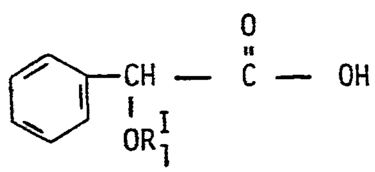

VII

wherein $R_1^I$ has the above meaning.

6. 7-(D-2-formyloxy-2-phenyl)acetamido-3-[(1-sulfomethyltetrazol-5-yl)-thiomethyl]-3-cephem-4-carboxylic acid and salts thereof.

7. A compound according to Claim 6 in disodium salt form.